# EUROPEAN PATENT SPECIFICATION

(11) **EP 1 778 093 B1**
(45) Date of publication and mention of the grant of the patent: **03.04.2013**
(21) Application number: 05780735.6
(22) Date of filing: 19.07.2005
(51) Int. Cl.: A61B 8/08, A61B 8/14, G06T 7/00, G06T 7/20, G01S 15/89, G06T 5/00, G01S 7/52

(54) **ULTRASONIC DIAGNOSIS OF ISCHEMIC CARDIODISEASE**
ULTRASCHALLDIAGNOSE EINER ISCHÄMISCHEN HERZERKRANKUNG
DIAGNOSTIC ULTRASONORE DE MALADIE CARDIAQUE ISCHEMIQUE

(30) Priority: 11.08.2004 US 600486 P
(43) Date of publication of application: 02.05.2007
(73) Proprietor: Koninklijke Philips Electronics N.V., 5621 BA Eindhoven (NL)
(72) Inventor: SALGO, Ivan, Bothell, WA 98041-3003 (US); DAVENPORT, Andrew, Bothell, WA 98041-3003 (US); KELTON, William, Bothell, WA 98041-3003 (US)
(74) Representative: Verweij, Petronella Danielle
(86) International application number: PCT/IB2005/052418
(87) International publication number: WO 2006/024970

(56) References cited:
- US-A- 5 241 473
- US-A- 5 533 510
- US-A- 5 797 843
- US-A- 5 860 927
- US-A1- 2002 072 671
- SANCHEZ-ORTIZ G I ET AL: "Automating 3D echocardiographic image analysis" MEDICAL IMAGE COMPUTING AND COMPUTER-ASSISTED INTERVENTION - MICCAI 2000. THIRD INTERNATIONAL CONFERENCE. PROCEEDINGS (LECTURE NOTES IN COMPUTER SCIENCE VOL.1935) SPRINGER-VERLAG BERLIN, GERMANY, 2000, pages 687-696, XP019001315 ISBN: 3-540-41189-5
- WILSON D C ET AL: "Automated analysis of echocardiographic apical four-chamber images" PROCEEDINGS OF THE SPIE - THE INTERNATIONAL SOCIETY FOR OPTICAL ENGINEERING SPIE-INT. SOC. OPT. ENG USA, vol. 4121, 2000, pages 128-139, XP002357973 ISSN: 0277-786X cited in the application

## Description

This invention relates to ultrasonic diagnostic imaging systems and, in particular, to ultrasonic imaging diagnosis of ischemic cardiac disorders.

The present invention relates to an ultrasonic diagnosis apparatus and method in which movement of an organ in motion, such as the cardiac muscle (myocardium) of a heart, is obtained and displayed and if necessary, on the basis of the movement, diagnosis of ischemic and other functional disorders is performed. In particular, the ultrasonic diagnostic apparatus and method relate to an apparatus and method effective in diagnosis of ischemic cardiac diseases such as myocardial ischemia and angina pectoris, left ventricular distention disorders including hypertrophic cardiomyopathy, and disorders of the conducting system of the heart such as Wolff-Parkinson-White syndrome and left bundle branch block.

In diagnosis of the above-mentioned ischemic cardiac diseases; left ventricular distention disorders and disorders of the conducting system of the heart are of considerable interest. But with conventional B mode imaging it is very difficult to acquire detailed information with respect to detection of local deteriorated portions in contraction ability in ischemic cardiodisease, objective diagnosis of left ventricle distention disorders, and detection of the positions and extent of abnormal paries movement in a conducting system of the heart.

One approach to overcoming this difficulty is an analytical method of paries movement of the left ventricle. This method measures changes in thickness of the cardiac muscle of the left ventricle at both systole and diastole and concludes that a region of lesser change in thickness is a region of reduced contraction ability or ischemic region. There have been various algorithms proposed for this method which generally require tracing the endocardium or the epicardium of the left ventricle in both end-systole and end-diastole views on B-mode tomographic images.

Stress echography is also known for diagnosing myocardial ischemia. Carrying out a stress echography procedure requires a heart to be stressed by exercise, drugs or an electric stimulus. B mode tomographic images of the heart are recorded before and after stressing, respectively, and displayed side-by-side in comparison. Changes in thickness of the cardiac muscle are compared in systolic and diastolic views (normally, thicker in systole) to detect a region of myocardial infarction. It is also generally required for this detection to trace the inner and outer walls and the center line of the cardiac muscle on the images to define the contour of the myocardium.

US patent 5,241,473 (Ishihara et al.) describes a method for forming differential image data by performing differential operation among topographic image data obtained in time series and an apparatus for giving color codes which designate display colors to differential image data. It further discloses an apparatus for cumulating a plurality of frames of differential image data, and an apparatus for displaying respective cumulated differential images in colors designated by the color codes in which motion of a moving portion of a patient's body is displayed in colors by superposing a plurality of differential images.

G.I. Sanchez-Ortiz et al. "Automating 3D echocardiographic image analysis" (appeared in Medical Image Computing and Computer-Assisted Intervention - MICCAI 2000. Third International Conference. Proceedings LNCS Vol. 1935, Springer Verlag, Berlin, Germany, 2000, pages 687-696) presents a fully-automated 3D echocardiographic image processing protocol for assessment of left ventricular (LV) function.

A number of automated and semi-automated techniques have been developed for defining the myocardium by tracing its boundaries in an ultrasound image. For example, US patent 6,491,636 (Chenal et al.) describes a technique for automatically tracing the endocardial border of the left ventricle of the heart which uses corner templates and septal wall angle bisection to geometrically identify the medial mitral annulus, the lateral mitral annulus and the apex of the left ventricle, then fits a border template to the three identified landmarks in the image. US patent 6,346,124 (Geiser et al.) traces both the endocardial border and the epicardial border by image analysis using expert reference echocardiographic image borders. See also US patent 5,797,396 (Geiser et al.) which describes a technique for identifying elliptical borders in ultrasound images. Another automated border tracing technique is described in US patent application number US 2005/0075567 A1. In this technique a user begins by delineating first and second landmarks on a tissue boundary of a diagnostic image such as the medial and lateral mitral annuli of the left ventricular endocardium. The user then delineates a third landmark on the tissue boundary such as the ventricular apex and a processor then fits a border template to this first tissue boundary, the endocardium. The user delineates a fourth landmark on another boundary of the tissue such as the epicardium and the processor fits a second border template to the second tissue boundary. The template shapes can then be adjusted by the user to precisely match the epicardial and endocardial boundaries.

The robustness of the tracing technique is ultimately determined by the quality of the image, however. Cardiac imaging can pose a number of challenges to image quality. The heart is enclosed in the rib cage which limits the acoustic windows available for cardiac imaging. The heart is often scanned from below the ribs with the heart viewed from the apex, requiring the ultrasound to penetrate through and return from a considerable distance into the body. Such apical views cause the beam directions to be almost parallel to the lateral wall of the left ventricle rather than orthogonal which would return the strongest echoes. The endocardial lining is a delicate tissue which often is not a strong reflector of ultrasound energy. And of course, the heart is in constant motion. Consequently, the endocardial border cannot always be traced with utmost confidence. Accordingly it is desirable to provide diagnostic techniques for ischemic and arterial cardiodiseases which can assess left ventricular infirmities without the need to continually define the endocardial border.

In accordance with the principles of the present invention, an ultrasonic diagnostic apparatus and technique are provided for diagnosing ischemic cardiac disorders. The mitral valve location is distinguished in a sequence of real time images of the left ventricle as it moves with expansion and contraction of the heart chamber. The valve location over at least a portion of the heart cycle is retained in the images such that the buildup of a sequence of successive valve locations is displayed. The variation in the changes in valve location reveal defects in conduction and motion of the heart wall. In accordance with a further aspect of the present invention the mitral valve location is distinguished by a representation of the mitral valve plane in a cross-sectional view of the valve.

In the drawings:
FIGURE 1 illustrates in block diagram form an ultrasonic diagnostic imaging system constructed in accordance with the principles of the present invention;
FIGURE 2 is an ultrasound image of the left ventricle in which the changing locations of the mitral valve plane are depicted in gradated color shadings;
FIGURES 3a and 3b illustrate the step of locating the medial mitral annulus (MMA) and the lateral mitral annulus (LMA) in an ultrasound image of the left ventricle (LV);
FIGURE 4 illustrates the step of locating the apex of the LV;
FIGURES 5a-5c illustrate standard border shapes for the LV;
FIGURES 6a-6b illustrate geometric templates used to locate the MMA and LMA;
FIGURES 7a-7c illustrate a technique for fitting a standard border shape to the endocardial boundary of the LV;
FIGURE 8 illustrates an end diastole and end systole display with endocardial borders drawn automatically;
FIGURE 9 illustrates in block diagram form a second embodiment of an ultrasonic diagnostic imaging system constructed in accordance with the principles of the present invention;
FIGURES 10A-10D are a sequence of images showing the tracing of a myocardial boundary in accordance with the principles of the present invention; and
FIGURES 11a-11c illustrate different progressions of locational change of the mitral valve plane which are characteristic of certain pathological conditions.

Physicians commonly must diagnose patients exhibiting symptoms of heart failure, constriction or restriction. Observing and measuring the motion of the heart muscle with ultrasound is routinely done by cardiologists in these situations. In conventional practice physicians examine both the systolic contraction and diastolic relaxation with spectral Doppler to analyze motion of the mitral annulus, the ring of leaflet attachment in the left ventricle (LV). This analysis can be used to estimate the timing and overall motion of the LV during contraction as well as understanding the nature of constrictive and restrictive diseases of the myocardium. For example, late contraction of the LV lateral wall results in delayed excursion of the mitral annulus on that side. The present invention describes apparatus and a method for detecting and quantifying these motional aberrations of a diseased heart. This invention describes the tracking of mitral annular motion for parametric display of mitral annular motion; use of this tracking information to map Doppler motion onto the parametric display; and to quantify both the timing and degree of excursion of mitral annular motion.

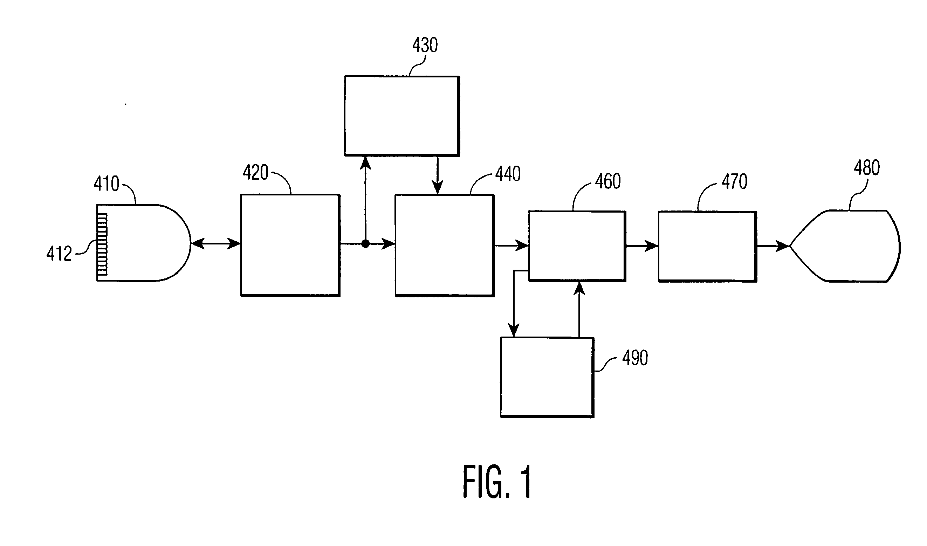

Referring now to FIGURE 1, a first embodiment of an ultrasonic diagnostic imaging system constructed in accordance with the principles of the present invention is shown in block diagram form. A probe or scanhead 410 which includes a one dimensional (1D) or two dimensional (2D) array 412 of transducer elements transmits ultrasonic waves and received ultrasonic echo signals. This transmission and reception is performed under control of a beamformer 420 which processes received echo signals to form coherent beams of echo signals from the anatomy being scanned. The echo information is Doppler processed by a Doppler processor 430 when Doppler information is to be presented, and the processed Doppler information is coupled to an image processor 440 which forms 2D or 3D Doppler images. For B mode imaging of tissue structure the echo signals are image processed by amplitude detection and scan converted into the desired image format for display. The images pass through a Cineloop memory 460 from which they may be coupled directly to a video processor 470 for display on an image display 480. The images may also be applied to an automatic border detection (ABD) processor 490 which operates on the 2D or 3D images to define the anatomical borders and boundaries in the images as described below. The defined borders are overlaid on the images which are coupled to the video processor 470 for display. The system may operate to define and display borders on loops of images saved in the Cineloop memory 460, or to display borders drawn on real time images produced during live scanning of a patient.

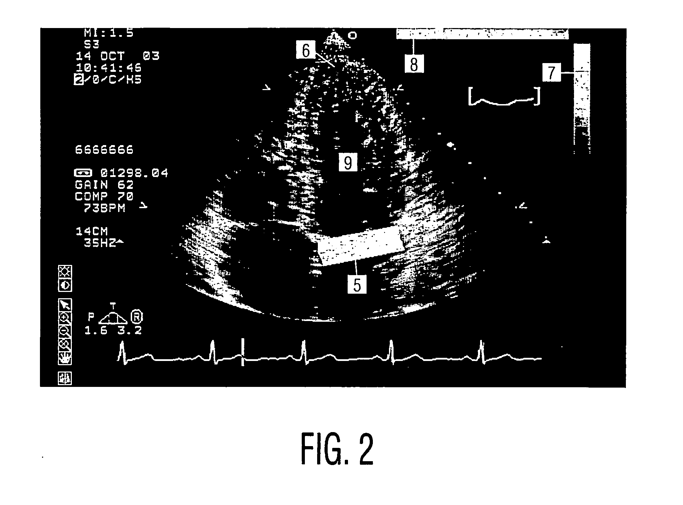

The ultrasound system of FIGURE 1 can be used to produce static or live images depicting mitral annular motion as shown in FIGURE 2, which is an image taken from a constructed embodiment of the present invention. Those skilled in the art will recognize the four chamber apical grayscale ultrasound image of a heart in the center of FIGURE 2 which shows all four chambers of the heart in cross-section in this two dimensional image. To the right of the ultrasound image is the standard grayscale bar 7 for the image showing the range of shading used in the image. This image is acquired by a probe 410 placed below the patient's ribs and directed upward toward the apex of the heart. The reference number 9 in FIGURE 2 marks the center of the LV with its apex 6 at the top of the ultrasound image. At the opposite side of the LV is the mitral valve. When the LV of a healthy heart contracts during the systolic phase of the heart cycle the myocardial walls of the LV all move smoothly and uniformly toward the center of the LV, including the side of the heart where the mitral valve is located. Thus, by this contractive action the mitral valve moves upward in the image toward the apex 6. During diastole the mitral valve moves back to its starting location as the heart muscle relaxes.

In accordance with the principles of the present invention the location of the mitral valve is tracked and depicted on the ultrasound image during the systolic phase, the diastolic phase, or both. A sequence of images acquired during a heart cycle are analyzed to detect the mitral valve annulus as described below or by other known techniques. Preferably the position of the mitral annulus is detected rather than the valve leaflets to provide a more stable motional reference. The mitral valve location is graphically marked on an image as by a distinctive line or color stripe. This process is repeated for the next and all successive images in the sequence. Furthermore, the lines or stripes are accumulated so that each new image retains the lines or stripes identified in the previous images in the sequence and in the same locations in relation to a static reference in which they were detected. As the sequence progresses the lines or stripes build up, depicting the path of successive positions of the mitral valve during the sequence of contraction or expansion. A build-up 5 of such color stripes is shown in FIGURE 2. In the actual color image from which FIGURE 2 is reproduced the build-up of stripes changes hue from orange to yellow to green, in correspondence with the color bar 8 at the top of the display. The variations in hues or shadings of the color bar can be based upon various quantification metrics. For example, the mitral valve location of each successive image can be assigned a successive different hue or shade. Thus, each image frame in the sequence uses a successively different hue or shade. Alternatively, each successive hue or shade can correspond to a particular increment of motion such as 0.XX mm. In this embodiment a wide range of colors indicates a large range of motion as the spread of the line or strip build-up shows. As a third alternative, each successive hue or color can represent an increment in time during the heart cycle. Such a gradation can be synchronized to the frame acquisition times, for instance.

Each time the predetermined heart phase or phases have completed and the mitral valve motion 5 depicted for that heart cycle interval has been fully depicted, the build-up of lines or stripes is deleted until the predetermined phase starts again during a successive heart cycle. If the user decides to depict the mitral valve motion during systole the first line or stripe will be drawn at a lower position on the display and continually move upward as the heart contracts and the mitral valve moves toward the apex of the heart. If the user decides to depict mitral valve motion during diastole the lines or stripes will begin at a higher position on the display and progressively build up toward the bottom of the screen as the heart muscle relaxes and the mitral valve location moves away from the apex. If both heart phases are chosen the build-up of colors or shades will alternately move upward and then downward on the screen.

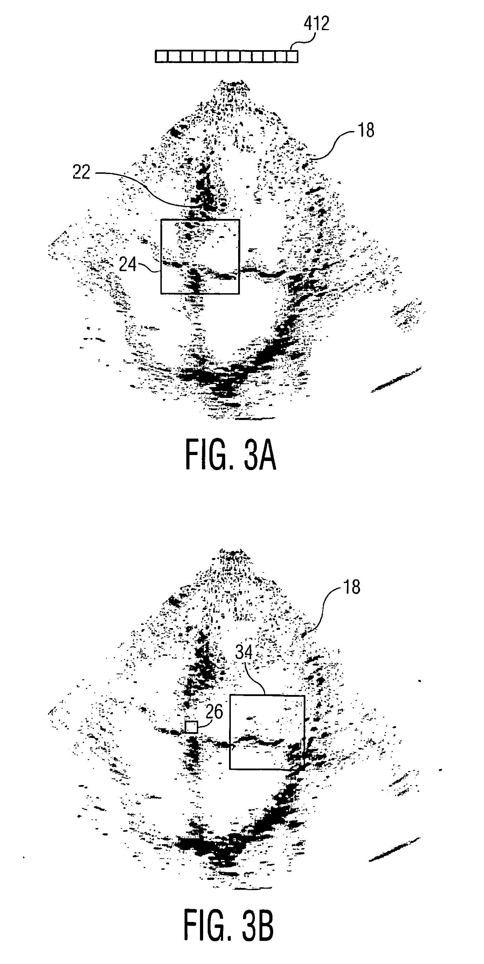

One technique for detecting the location of the mitral valve in a sequence of heart images is shown starting with FIGURES 3a and 3b. In this example the ABD processor 490 begins by identifying and tracing the mitral valve plane in the LV in the end systole image 18. The first step in tracing the mitral valve plane of the LV is to locate two key landmarks in the image, the medial mitral annulus (MMA) and the lateral mitral annulus (LMA). This process begins by defining a search area for the MMA as shown in FIGURE 3a, in which the ultrasound image grayscale is reversed from white to black (and black to white) for ease of illustration. Since the ABD processor is preconditioned in this example to analyze four-chamber views of the heart with the transducer array 412 viewing the heart from its apex, the processor expects the brightest vertical nearfield structure in the center of the image to be the septum which separates the left and right ventricles. This means that the column of pixels in the image with the greatest total brightness value should define the septum. With these cues the ABD processor locates the septum 22, and then defines an area in which the MMA should be identified. This area is defined from empirical knowledge of the approximate depth of the mitral valve from the transducer in an apical view of the heart. A search area such as that enclosed by the box 24 in FIGURE 3a is defined in this manner.

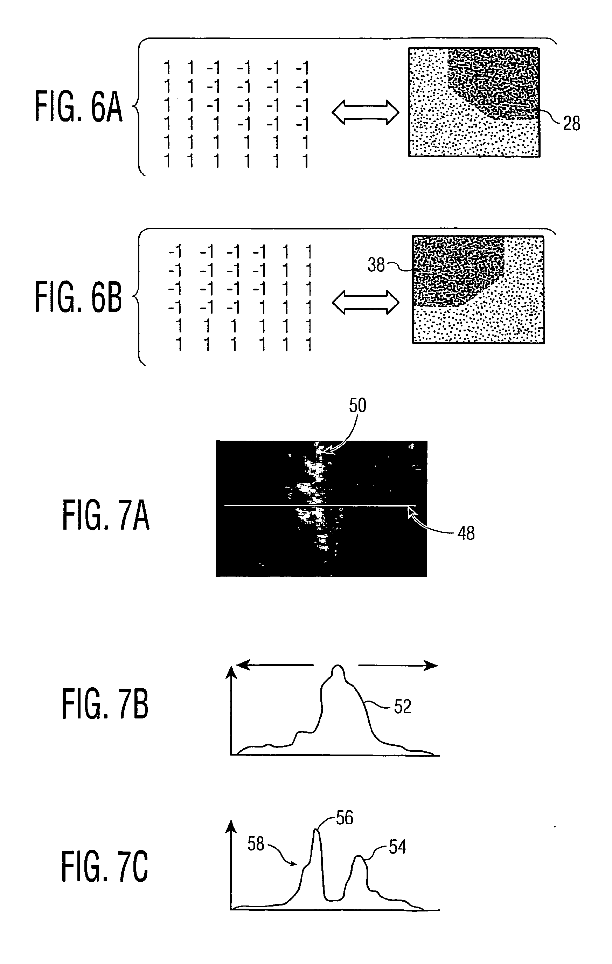

A filter template defining the anticipated shape of the MMA is then cross-correlated to the pixels in the MMA search area. While this template may be created from expert knowledge of the appearance of the MMA in other four-chamber images as used by Wilson et al. in their paper "Automated analysis of echocardiographic apical 4-chamber images," Proc. of SPIE, August, 2000, the illustrated example uses a geometric corner template. While a right-angle corner template may be employed, in a constructed embodiment an octagon corner template 28 (the lower left corner of an octagon) is used as the search template for the MMA, as shown at the right side of FIGURE 6a. In practice, the octagon template is represented by the binary matrix shown at the left side of FIGURE 6a. The ABD processor performs template matching by cross correlating different sizes of this template with the pixel data in different translations and rotations until a maximum correlation coefficient above a predetermined threshold is found. To speed up the correlation process, the template matching may initially be performed on a reduced resolution form of the image, which highlights major structures and may be produced by decimating the original image resolution. When an initial match of the template is found, the resolution may be progressively restored to its original quality and the location of the MMA progressively refined by template matching at each resolution level.

Once the MMA has been located a similar search is made for the location of the LMA, as shown in FIGURE 3b. The small box 26 marks the location previously established for the MMA in the image 18, and a search area to the right of the MMA is defined as indicated by the box 34. A right corner geometric template, preferably a right octagon corner template 38 as shown in FIGURE 6b, is matched by cross-correlation to the pixel values in the search area of box 34. Again, the image resolution may be decimated to speed the computational process and different template sizes may be used. The maximal correlation coefficient exceeding a predetermined threshold defines the location of the LMA.

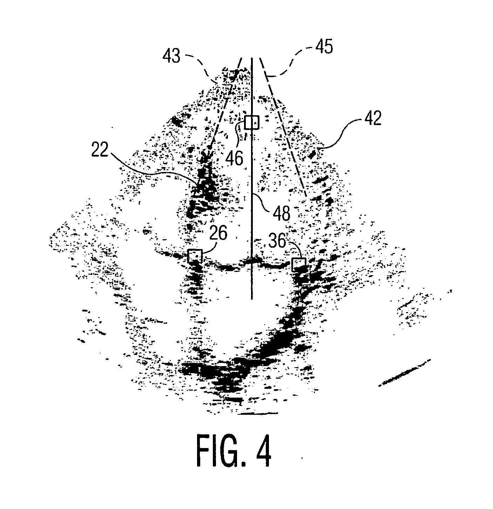

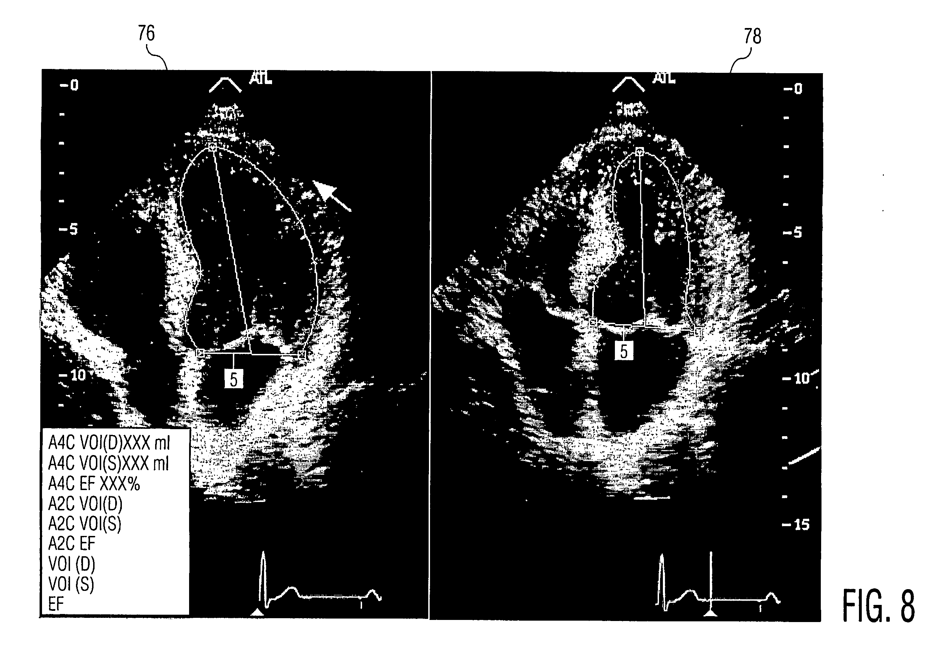

With the MMA 26 and the LMA 36 found as shown in FIGURE 4, these two points may be connected by displaying a line 5 between the two points as shown in FIGURE 8. The line 5 may be colored or shaded in accordance with the gradation of a color bar 8 as previously described. This process is repeated to identify the mitral valve plane in each of the successive images, and the build-up of lines 5 displayed as described above.

This technique for identifying the mitral valve plane may be continued to define the full endocardial border as follows. While this continuation is not necessary in an implementation of the present invention, and may in fact be undesired for the additional graphical complexity it introduces into the images, it may be desired for further diagnostic purposes such as producing a color representation of LV wall motion known as color kinesis and described in US Pat. 5,533,510 (Koch, III et al.)

To trace the full endocardial border an additional landmark, the endocardial apex, is found. The position of the endocardial apex may be determined as shown in FIGURE 4. The pixel values of the upper half of the septum 22 are analyzed to identify the nominal angle of the upper half of the septum, as indicated by the broken line 43. The pixel values of the lateral wall 42 of the LV are analyzed to identify the nominal angle of the upper half of the lateral wall 42, as shown by the broken line 45.
If the lateral wall angle cannot be found with confidence, the angle of the scanlines on the right side of the sector is used. The angle between the broken lines 43,45 is bisected by a line 48, and the apex is initially assumed to be located at some point on this line. With the horizontal coordinate of the apex defined by line 48, a search is made of the slope of pixel intensity changes along the line 48 to determine the vertical coordinate of the apex. This search is made over a portion of line 48 which is at least a minimum depth and not greater than a maximum depth from the transducer probe, approximately the upper one-quarter of the length of line 48 above the mitral valve plane between the MMA 26 and the LMA 36. Lines of pixels along the line 48 and parallel thereto are examined to find the maximum positive brightness gradient from the LV chamber (where there are substantially no specular reflectors) to the heart wall (where many reflectors are located). A preferred technique for finding this gradient is illustrated in FIGURE 7. FIGURE 7a shows a portion of an ultrasound image including a section of the heart wall 50 represented by the brighter pixels in the image. Drawn normal to the heart wall 50 is a line 48 which, from right to left, extends from the chamber of the LV into and through the heart wall 50. If the pixel values along line 48 are plotted graphically, they would appear as shown by curve 52 in FIGURE 7b, in which brighter pixels have greater pixel values. The location of the endocardium is not the peak of the curve 52, which is in the vicinity of the center of the heart wall, but relates to the sense of the slope of the curve. The slope of the curve 52 is therefore analyzed by computing the differential of the curve 52 as shown by the curve 58 in FIGURE 7c. This differential curve has a peak 56 which is the maximal negative slope at the outside of the heart wall (the epicardium). The peak 54, which is the first major peak encountered when proceeding from right to left along curve 58, is the maximal positive slope which is the approximate location of the endocardium. The pixels along and parallel to line 48 in FIGURE 4 are analyzed in this manner to find the endocardial wall and hence the location of the endocardial apex, marked by the small box 46 in FIGURE 4.

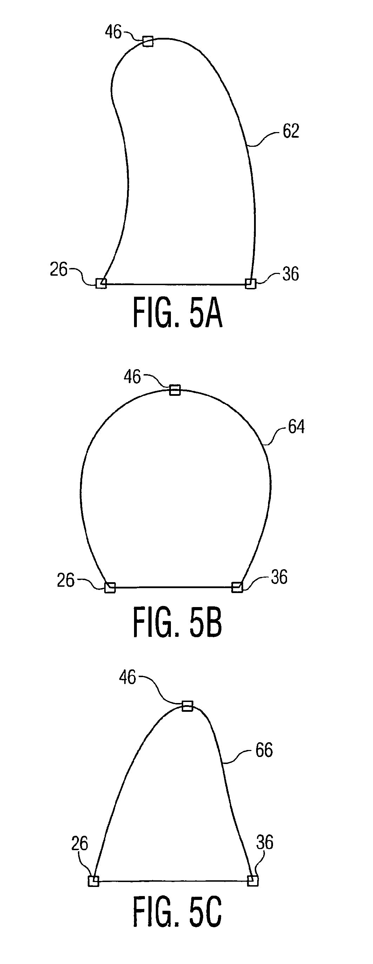

Once these three major landmarks of the LV have been located, one of a number of predetermined standard shapes for the LV is fitted to the three landmarks and the endocardial wall. Three such standard shapes are shown in FIGURES 5a, 5b, and 5c. The first shape, border 62, is seen to be relatively tall and curved to the left. The second shape, border 64, is seen to be relatively short and rounded. The third shape, border 66, is more triangular. Each of these standard shapes is scaled appropriately to fit the three landmarks 26,36,46. After an appropriately scaled standard shape is fit to the three landmarks, an analysis is made of the degree to which the shape fits the border in the echo data. This may be done, for example, by measuring the distances between the shape and the heart wall at points along the shape. Such measurements are made along paths orthogonal to the shape and extending from points along the shape. The heart wall may be detected using the operation discussed in FIGURES 7a-7c, for instance. The shape which is assessed as having the closest fit to the border to be traced, by an average of the distance measurements, for instance, is chosen as the shape used in the continuation of the process.

The chosen shape is then fitted to the border to be traced by "stretching" the shape, in this example, to the endocardial wall. The stretching is done by analyzing 48 lines of pixels evenly spaced around the border and approximately normal to heart wall. The pixels along each of the 48 lines are analyzed as shown in FIGURES 7a-7c to find the adjacent endocardial wall and the chosen shape is stretched to fit the endocardial wall. The baseline between points 26 and 36 is not fit to the shape but is left as the straight line previously found for the nominal plane of the mitral valve. When the shape has been fit to points along the heart wall, the border tracing is smoothed and displayed over the end systole image as shown in the image 78 on the right side of the dual display of FIGURE 8. The display includes five control points shown as X's along the border between the MMA landmark and the apex, and five control points also shown as X's along the border between the apex landmark and the LMA landmark. In this example the portion of line 48 between the apex and the mitral valve plane is also shown, as adjusted by the stretching operation.

With the end systole border drawn in this manner the ABD processor 490 now proceeds to determine the end diastole border when the end diastole image is in the sequence. It does so, not by repeating this operation on the end diastole image 16, but by finding a border on each intervening image in sequence between end systole and end diastole (or *vice versa).* In a given image sequence this may comprise 20-30 image frames. Since this is the reverse of the sequence in which the images were acquired, there will only be incremental changes in the endocardial border location from one image to the next. It is therefore to be expected that there will be a relatively high correlation between successive images. Hence, the end systole border is used as the starting location to find the border for the previous image, the border thus found for the previous image is used as the starting location to find the border for the next previous image, and so forth. In a constructed embodiment this is done by saving a small portion of the end systole image around the MMA and the LMA and using this image portion as a template to correlate and match with the immediately previous image to find the MMA and the LMA locations in the immediately previous image. The apex is located as before, by bisecting the angle between the upper portions of the septum and lateral LV wall, then locating the endocardium by the maximum slope of the brightness gradient. Since the LV is expanding when proceeding from systole to diastole, confidence measures include the displacement of the landmark points in an outward direction from frame to frame. When the three landmark points are found in a frame, the appropriately scaled standard shape is fit to the three points. Another confidence measure is distention of the standard shapes; if a drawn LV border departs too far from a standard shape, the process is aborted.

Border delineation continues in this manner until the end diastole image is processed and its endocardial border defined. The dual display then appears as shown in FIGURE 8, with endocardial borders drawn on both the end diastole and end systole images 76,78.

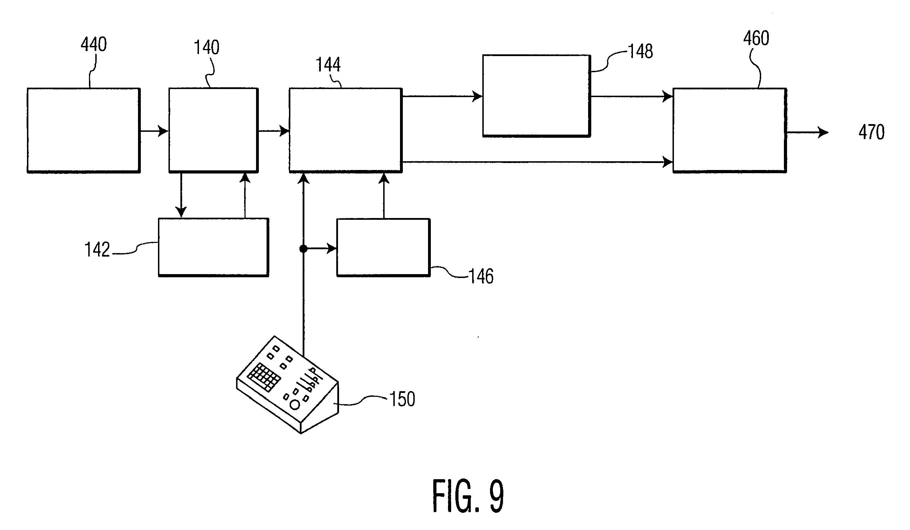

As FIGURE 8 shows, the endocardial borders of both the end diastole and end systole images have small boxes denoting the three major landmarks and control points marked by X's on the septal and lateral borders. The clinician chooses the default number of control point which will be displayed initially; on the border 80 shown in FIGURE 9 there are three control points shown on the septal wall and four control points shown on the lateral wall. The clinician can review the end diastole and systole images, as well as all of the intervening images of the loop if desired, and manually adjust the positions of the landmark boxes and control point X's if it is seen that the automated process placed a border in an incorrect position. The clinician can slide a box or X along the border to a new position, and can add more control points or delete control points from the border. The process by which the clinician relocates a box or X laterally is known as rubberbanding. Suppose that the ABD processor had initially located the control point and border at a position which the clinician observes is incorrect. The clinician can relocate the control point laterally by dragging the X with a screen pointing device to a new lateral location. As the X is dragged, the border moves or stretches along with the X, thereby defining a new border. In this manner the clinician can manually correct and adjust the borders drawn by the ABD processor. As the clinician laterally relocates a control point X, the ABD processor responds by automatically recalculating the positions of the adjoining border and adjacent control points if necessary so that the border remains smoothly continuous. The recalculation will not adjust the position of a control point or landmark box which has been previously manually repositioned by the clinician, thereby preserving this expert input into the border drawing process. If the clinician relocates a landmark box, the ABD processor recalculates and refits the entire border to the landmarks and heart wall. Since the adjustment of one border in the image sequence can affect the borders of temporally adjacent images in the sequence, the ABD processor will also respond to a manual adjustment by correlating the adjusted border with temporally adjacent borders so that the manual adjustment is properly continuously represented in some or all of the images in the loop.

Further details of this endocardial border technique may be found in US Pat. 6,491,636 (Chenal et al.)

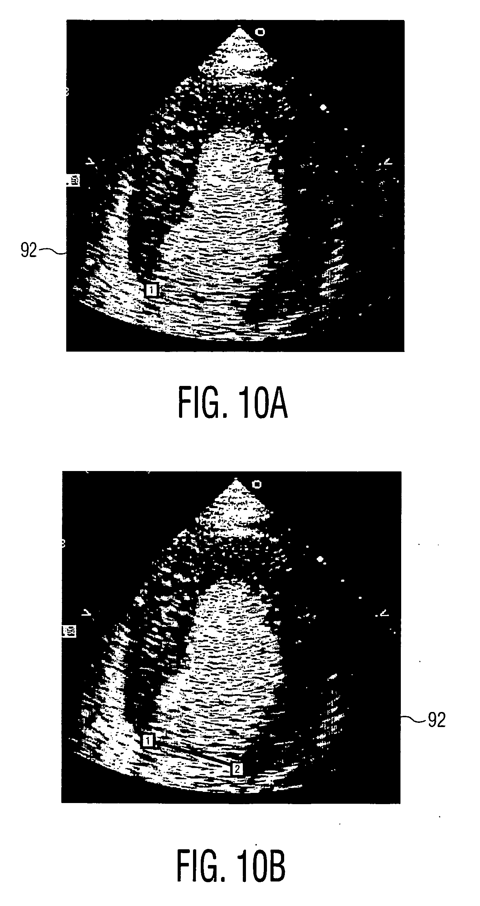

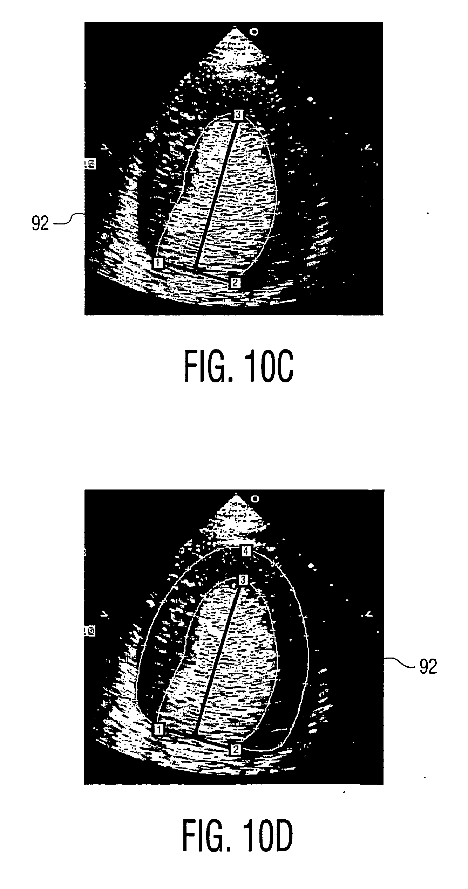

A second embodiment for identifying the location of the mitral valve is illustrated in FIGURES 9 and 10. Echo signals processed by the image processor 440 are stored in an image data memory 140. The image data used for an image is forwarded to a scan converter 142 which produces image data of the desired image format, e.g., sector, rectangular, virtual apex, or curved linear. The scan converted image data is stored in the image data memory from which it is accessed by an assisted border detector 144. The assisted border detector 144 is responsive to input from a user control such as the trackball pointing device on a user control panel 150 to locate the control points with reference to the image data and position and stretch the standard endocardial shape with respect to the image data. The standard shape data is provided by a border template storage device 146. As the control points and borders are being drawn and positioned on the image, the control point and border data produced by the assisted border detector 144 is applied to a border graphics processor 148, which produces a graphic overlay of the control points and border to be displayed with the image data. The graphic overlay and the image data are stored in a display memory such as the Cineloop memory 460, from which they are accessed for display by the video processor 470.

In accordance with the principles of another embodiment of the present invention, the mitral valve plane of the left ventricle is delineated by an assisted border detection technique as follows. The user displays an image 92 on which the mitral valve plane is to be located as shown in FIGURE 10A. The user designates a first landmark in the image with a pointing device such as a mouse or a trackball on the system control panel 150 which manipulates a cursor over the image. In the example of FIGURE 10A, the first landmark designated is the MMA. When the user clicks on the MMA in the image, a graphic marker appears such as the white control point indicated by the number "1" in the drawing. The user then designates a second landmark, in this example the LMA, which is marked with the second white control point indicated by the number "2" in FIGURE 10B. A line then automatically connects the two control points, which in the case of this longitudinal view of the left ventricle indicates the mitral valve plane. With the mitral valve plane thus defined by user assistance in one image, the mitral valve plane can then be confidently located in successive images in the image sequence by automated means as described in the previous embodiment. As discussed above, this can start with the use of the pixels of the MMA and LMA regions of image 92 as templates to find the MMA and LMA in temporally successive images. The mitral valve plane is thus identified automatically in the other images in the sequence and colored or shaded to produce the desired progression of the mitral valve location during designated phases of the heart cycle.

As in the previous embodiment, the process used to define the mitral valve plane can be continued to trace the full endocardial border. After identifying the MMA and LMA control points in the image 92, the user then moves the pointer to the endocardial apex, which is the uppermost point within the left ventricular cavity. As the user moves the pointer to this third landmark in the image, a template shape of the left ventricular endocardial cavity dynamically follows the cursor, distorting and stretching as the pointer seeks the apex of the chamber. This template, shown as a white line in FIGURE 10C, is anchored by the first and second control points 1 and 2 and passes through the third control point, which is positioned at the apex when the user clicks the pointer at the apex, thereby anchoring the third control point 3. When positioned, the endocardial cavity template provides an approximate tracing of the endocardium as shown in FIGURE 10C. In the embodiment of FIGURE 10C a black line which bisects the left ventricle follows the pointer as it approaches and designates the apex. This black line is anchored between the center of the line indicating the mitral valve plane and the left ventricular apex, essentially indicating a center line between the center of the mitral valve and the apex of the cavity.

With the endocardial border thus defined, the user can continue to define the epicardial border. The user moves the cursor to the epicardial apex, the uppermost point on the outer surface of the myocardium. The user then clicks on the epicardial apex and a fourth control point marked "4" is positioned. A second template then automatically appears which approximately delineates the epicardial border as shown in FIGURE 10D. This second template, shown by the outer white border line in FIGURE 10D, is also anchored by the first and second control points and passes through the positioned fourth control point at the epicardial apex. The two templates are an approximate outline of the complete myocardial border. As in the previous embodiment, control points are located around the border tracing which can be "grabbed" by the pointer and dragged in a rubberbanding operation to manually refine the border tracing.

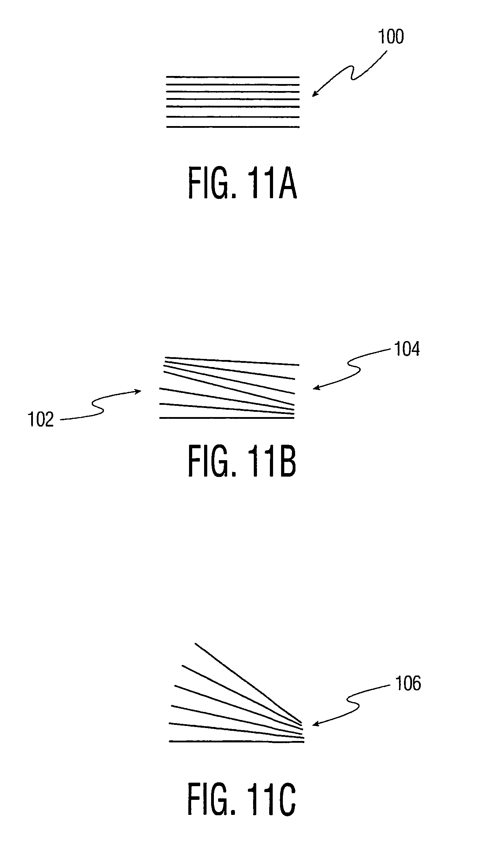

FIGURES 11a-11c illustrates three different build-ups of mitral valve location lines which may be encountered in various diagnoses. FIGURE 11a illustrates with a plurality of lines 100 the positions of the mitral valve location at successive moments during systole as the heart muscle contracts. In a healthy, normal heart the myocardium will contract uniformly in both time and space. When the mitral valve is at the bottom of the image as it is in FIGURE 2, this smooth, uniform contraction will lift the mitral valve upward toward the center of the LV and toward the apex of the chamber. As it does so the successive positions of the mitral valve will appear as a succession of substantially parallel edges or lines as shown in FIGURE 11a.

A diseased heart may be afflicted by a conduction delay on one side of the heart relative to another. When the heart contracts the myocardium should conduct the contractive motion instantaneously throughout the heart muscle. An abnormal heart may exhibit a delay in this contractive motion in a particular region of the heart. FIGURE 11b illustrates a conduction delay known as left bundle branch block which would cause late conduction on the right side of the heart as seen in FIGURE 2. A left bundle branch block will result in one side of the mitral valve plane moving faster initially than the other side. The other side of the mitral valve plane will move up later, resulting in the mitral valve location pattern shown in FIGURE 11b. The first series of mitral valve locations is seen to move faster on the left side as seen by lines 102. After this motion begins, the right side of the mitral valve plane will move upward as shown by the upper lines 104 in the sequence. The color-coding of the locations discussed above will produce a double wedge of color changes as the pattern of FIGURE 11b indicates.

If the patient has suffered a myocardial infarction (heart attack) one side of the mitral valve plane can appear to hardly move at all. Such a condition is illustrated by FIGURE 11c, which is the sequence of lines that would appear if the lateral wall of the heart seen in FIGURE 2 had suffered an infarction. The series of lines 106 would appear when the lateral wall has been infarcted to a degree that it is virtually stationary, resulting in most of the motion of the mitral valve plane in the image to appear at the septal wall side of the valve plane.

It is thus seen that the technique of the present invention can detect abnormal heart conditions even when the endocardial border is indistinct and too faint to be accurately traced.
For instance, the white arrow in the end diastole image on the left side of FIGURE 8 is pointing to the lateral wall of the LV which, for the reasons given above, appears very poorly defined in the image. Wall motion can be difficult to discern and trace accurately when the lateral wall is so poorly defined. However the effect of an abnormality of the lateral wall may be seen in its effect on the motion of the mitral valve plane during contraction and relaxation of the heart, enabling diagnosis in a difficult-to-image patient.

The technique of the present invention may be extended to three dimensional imaging. In 3D imaging the entire mitral valve location can be visualized, not just a cross-section as shown in the preceding examples. Depending on the graphical object in which it is decided to represent the valve plane location, the motional graphic may appear as a growing cylinder, cube, or other shape, and may be shaded or color-coded as described above. In a healthy heart the object will grow uniformly in shape but in a diseased heart the shape may appear nonuniform with a sloped or slanted surface or coloring. The tissue of the heart can be made semi-transparent so as to better visualize the mitral valve location graphic within the 3D image of the heart. Shading the graphic can cause the graphic to appear more distinct within the anatomy.

It will also be apparent to those skilled in the art that quantified numerical measures or representations of the excursions of the mitral valve annulus can be derived from the color coding or spacing of the successive mitral valve location lines or surfaces. Both valve positions and rates of change in position (derivatives of positional change or velocity) can be displayed to assist in the diagnosis. By restricting the motional graphic to the mitral valve location rather than the full endocardial border as is done in color kinesis, the clinician is provided with a relatively uncluttered image sequence from which to make a diagnosis.

## Claims

1. A method for depicting the functioning of the heart from a sequence of images acquired during a phase of the heart cycle comprising:
acquiring a sequence of images of the heart during a selected phase or phases of the heart cycle, which images include the mitral valve;
identifying the location of the mitral valve in the images of the sequence; and
displaying in a single heart image the locations of successive positions of the mitral valve during the sequence of images.

2. The method of Claim 1, wherein displaying further comprises displaying in a single heart image the distinctively differently displayed locations of successive positions of the mitral valve.

3. The method of Claim 2, wherein identifying further comprises identifying the location of the mitral valve annulus in the images; and
wherein displaying further comprises displaying in a single heart image the locations of successive positions of the mitral valve annulus.

4. The method of Claim 3, wherein identifying comprises identifying the location of the mitral valve annulus as shown in cross-section in a sequence of two dimensional images.

5. The method of Claim 3, wherein identifying comprises identifying the location of the mitral valve annulus as shown in a sequence of three dimensional images.

6. The method of Claim 2, wherein identifying further comprises identifying the location of the mitral valve plane in the images; and
wherein displaying further comprises displaying in a single heart image the locations of successive positions of the mitral valve plane.

7. The method of Claim 6, wherein identifying comprises identifying the location of the mitral valve plane as shown in cross-section in a sequence of two dimensional images.

8. The method of Claim 6, wherein identifying comprises identifying the location of the mitral valve plane as shown in a sequence of three dimensional images.

9. The method of Claim 2, wherein displaying further comprises displaying in a single heart image the distinctively differently shaded locations of successive positions of the mitral valve.

10. The method of Claim 2, wherein displaying further comprises displaying in a single heart image the distinctively differently colored locations of successive positions of the mitral valve.

11. The method of Claim 1, wherein displaying further comprises displaying in each of a plurality of images of the sequence the location of the mitral valve in that image and the location of the mitral valve in previous images in the sequence.

12. The method of Claim 11, further comprising resetting the display of a plurality of locations of the mitral valve following the end of the selected phase or phases of the heart cycle; and
repeating the acquiring, identifying and displaying steps during another heart cycle.

13. The method of Claim 1, wherein identifying further comprises manually identifying at least one reference point of the mitral valve in an acquired heart image.

14. The method of Claim 1, wherein identifying further comprises identifying the location of the mitral valve by automated border detection processing of the images.

15. The method of Claim 1, wherein displaying further comprises displaying in a single heart image the locations of successive positions of the mitral valve during the sequence of images in the absence of the display of successive positions of at least a portion of the endocardial wall, whereby image clutter is reduced.

16. An ultrasonic diagnostic imaging system for analyzing the performance of the heart comprising:
a probe (410) having a plurality (412) of transducer elements;
an image processor (440) coupled to the probe (410) which acts to produce a sequence of B mode images;
an automated border detection processor (490), which operates to identify the location of the mitral valve in a sequence of B mode images;
a graphic display processor (470), responsive to the identification of the location of the mitral valve in a sequence of B mode images, which act to produce a graphic depicting the locations of the mitral valve in a sequence of B mode images; and
a display (480) responsive to the graphic display processor which acts to display a B mode image with a graphic depicting the locations of the mitral valve in a previous sequence of images.

17. The ultrasonic diagnostic imaging system of Claim 16, further comprising a Cineloop memory (460), coupled to the automated border detection processor (490), which stores a sequence of B mode images.

18. The ultrasonic diagnostic imaging system of Claim 16, wherein the graphic display processor (470) further comprises a processor which acts to produce a graphic depicting the locations of the mitral valve in a sequence of B mode images by distinctive colors.

19. The ultrasonic diagnostic imaging system of Claim 16, wherein the automated border detection processor (490) further comprises a processor which operates to identify the location of the mitral valve plane in a sequence of B mode images.

20. An ultrasonic diagnostic imaging system for analyzing the performance of the heart comprising:
a probe (410) having a plurality of transducer elements;
an image processor (440) coupled to the probe (410) which acts to produce a sequence of B mode images;
a user control by which a user can identify the location of the mitral valve in at least one of a sequence of B mode images;
a graphic display processor (470), responsive to the identification of the location of the mitral valve in a sequence of B mode images, which act to produce a graphic depicting the locations of the mitral valve in a sequence of B mode images; and
a display (480) responsive to the graphic display processor (470) which acts to display a B mode image with a graphic depicting the locations of the mitral valve in a previous sequence of images.

21. The ultrasonic diagnostic imaging system of Claim 20, further comprising a border detection processor (490), responsive to the user control, which acts to identify the mitral valve location in a sequence of B mode images.

## Patentansprüche

1. Verfahren zur Darstellung der Herzfunktionen ausgehend von einer Folge von Bildern, die während einer Phase des Herzzyklus erfasst wurden, wobei das Verfahren Folgendes umfasst:
- Erfassen einer Folge von Bildern des Herzens während einer ausgewählten Phase oder während ausgewählter Phasen des Herzzyklus, wobei die Bilder die Mitralklappe enthalten,
- Identifizieren der Lage der Mitralklappe in den Bildern der Folge und
- Anzeigen der Lagen aufeinanderfolgender Positionen der Mitralklappe während der Folge von Bildern in einem einzigen Bild des Herzens.

2. Verfahren nach Anspruch 1, wobei das Anzeigen ferner das Anzeigen der deutlich unterschiedlich angezeigten Lagen aufeinanderfolgender Positionen der Mitralklappe in einem einzigen Bild des Herzens umfasst.

3. Verfahren nach Anspruch 2, wobei das Identifizieren ferner das Identifizieren der Lage des Mitralklappenanulus in den Bildern umfasst, und
wobei das Anzeigen ferner das Anzeigen der Lagen aufeinanderfolgender Positionen des Mitralklappenanulus in einem einzigen Bild des Herzens umfasst.

4. Verfahren nach Anspruch 3, wobei das Identifizieren das Identifizieren der Lage des im Querschnitt in einer Folge von zweidimensionalen Bildern gezeigten Mitralklappenanulus umfasst.

5. Verfahren nach Anspruch 3, wobei das Identifizieren das Identifizieren der Lage des in einer Folge von dreidimensionalen Bildern gezeigten Mitralklappenanulus umfasst.

6. Verfahren nach Anspruch 2, wobei das Identifizieren ferner das Identifizieren der Lage der Mitralklappenebene in den Bildern umfasst, und
wobei das Anzeigen ferner das Anzeigen der Lagen aufeinanderfolgender Positionen der Mitralklappenebene in einem einzigen Bild des Herzens umfasst.

7. Verfahren nach Anspruch 6, wobei das Identifizieren das Identifizieren der Lage der im Querschnitt in einer Folge von zweidimensionalen Bildern gezeigten Mitralklappenebene umfasst.

8. Verfahren nach Anspruch 6, wobei das Identifizieren das Identifizieren der Lage der in einer Folge von dreidimensionalen Bildern gezeigten Mitralklappenebene umfasst.

9. Verfahren nach Anspruch 2, wobei das Anzeigen ferner das Anzeigen der deutlich unterschiedlich schattierten Lagen aufeinanderfolgender Positionen der Mitralklappe in einem einzigen Bild des Herzens umfasst.

10. Verfahren nach Anspruch 2, wobei das Anzeigen ferner das Anzeigen der deutlich unterschiedlich eingefärbten Lagen aufeinanderfolgender Positionen der Mitralklappe in einem einzigen Bild des Herzens umfasst.

11. Verfahren nach Anspruch 1, wobei das Anzeigen ferner das Anzeigen der Lage der Mitralklappe in diesem Bild und die Lage der Mitralklappe in vorherigen Bildern in der Folge in jedem einer Vielzahl von Bildern der Folge umfasst.

12. Verfahren nach Anspruch 11, das ferner das Zurücksetzen der Anzeige einer Vielzahl von Lagen der Mitralklappe nach dem Ende der ausgewählten Phase oder Phasen des Herzzyklus und
das Wiederholen der Schritte der Erfassung, Identifizierung und Anzeige während eines weiteren Herzzyklus umfasst.

13. Verfahren nach Anspruch 1, wobei das Identifizieren ferner das manuelle Identifizieren zumindest eines Bezugspunktes der Mitralklappe in einem erfassten Bild des Herzens umfasst.

14. Verfahren nach Anspruch 1, wobei das Identifizieren ferner das Identifizieren der Lage der Mitralklappe durch Verarbeitung mit automatischer Randerkennung der Bilder umfasst.

15. Verfahren nach Anspruch 1, wobei das Anzeigen ferner das Anzeigen der Lagen aufeinanderfolgender Positionen der Mitralklappe während der Folge von Bildern bei fehlender Anzeige aufeinanderfolgender Positionen zumindest eines Teilstückes der Endokardwand in einem einzigen Bild des Herzens umfasst, wodurch Bildstörungen reduziert werden.

16. Ultraschalldiagnose-Bildgebungssystem zur Analyse der Leistung des Herzens, das Folgendes umfasst:
- einen Schallkopf (410) mit einer Vielzahl (412) von Wandlerbauteilen,
- einen Bildprozessor (440), der mit dem Schallkopf (410) gekoppelt ist und eine Folge von B-Mode-Bildern erzeugt,
- einen Prozessor für automatische Randerkennung (490), der so funktioniert, dass er die Lage der Mitralklappe in einer Folge von B-Mode-Bildern identifiziert,
- einen Prozessor für graphische Anzeige (470), der auf die Identifizierung der Lage der Mitralklappe in einer Folge von B-Mode-Bildern reagiert, indem er eine Graphik erzeugt, die die Lagen der Mitralklappe in einer Folge von B-Mode-Bildern darstellt, und
- eine Anzeige (480), die auf den Prozessor für graphische Anzeige reagiert, indem sie ein B-Mode-Bild mit einer Graphik anzeigt, die die Lagen der Mitralklappe in einer vorherigen Folge von Bildern darstellt.

17. Ultraschalldiagnose-Bildgebungssystem nach Anspruch 16, das ferner einen Cineloop-Speicher (460) umfasst, der mit dem Prozessor für automatische Randerkennung (490) gekoppelt ist und eine Folge von B-Mode-Bildern speichert.

18. Ultraschalldiagnose-Bildgebungssystem nach Anspruch 16, wobei der Prozessor für graphische Anzeige (470) ferner einen Prozessor umfasst, der so funktioniert, dass er eine Graphik erzeugt, die die Lagen der Mitralklappe in einer Folge von B-Mode-Bildern durch unterschiedliche Farben darstellt.

19. Ultraschalldiagnose-Bildgebungssystem nach Anspruch 16, wobei der Prozessor für automatische Randerkennung (490) ferner einen Prozessor umfasst, der so funktioniert, dass er die Lage der Mitralklappenebene in einer Folge von B-Mode-Bildern identifiziert.

20. Ultraschalldiagnose-Bildgebungssystem zur Analyse der Leistung des Herzens, das Folgendes umfasst:
- einen Schallkopf (410) mit einer Vielzahl von Wandlerbauteilen,
- einen Bildprozessor (440), der mit dem Schallkopf (410) gekoppelt ist und eine Folge von B-Mode-Bildern erzeugt,
- ein benutzerseitiges Bedienelement, mit dem ein Benutzer die Lage der Mitralklappe in zumindest einem einer Folge von B-Mode-Bildern identifizieren kann,
- einen Prozessor für graphische Anzeige (470), der auf die Identifizierung der Lage der Mitralklappe in einer Folge von B-Mode-Bildern reagiert, indem er eine Graphik erzeugt, die die Lagen der Mitralklappe in einer Folge von B-Mode-Bildern darstellt, und
- eine Anzeige (480), die auf den Prozessor für graphische Anzeige (470) reagiert, indem sie ein B-Mode-Bild mit einer Graphik anzeigt, die die Lagen der Mitralklappe in einer vorherigen Folge von Bildern darstellt.

21. Ultraschalldiagnose-Bildgebungssystem nach Anspruch 20, das ferner einen Prozessor für automatische Randerkennung (490) umfasst, der auf das benutzerseitige Bedienelement reagiert und die Lage der Mitralklappe in einer Folge von B-Mode-Bildern identifiziert.

## Revendications

1. Procédé pour décrire le fonctionnement du coeur à partir d'une séquence d'images acquises au cours d'une phase du cycle cardiaque comprenant :
l'acquisition d'une séquence d'images du coeur au cours d'une phase ou de phases sélectionnée(s) du cycle cardiaque, lesquelles images comportent la valvule mitrale;
l'identification de l'emplacement de la valvule mitrale sur les images de la séquence; et
l'affichage sur une seule image du coeur des emplacements des positions successives de la valvule mitrale au cours de la séquence d'images.

2. Procédé selon la revendication 1, dans lequel l'affichage comprend en outre l'affichage sur une seule image du coeur des emplacements affichés différemment de façon distinctive des positions successives de la valvule mitrale.

3. Procédé selon la revendication 2, dans lequel l'identification comprend en outre l'identification de l'emplacement de l'anneau de la valvule mitrale sur les images ; et
dans lequel l'affichage comprend en outre l'affichage sur une seule image du coeur des emplacements des positions successives de l'anneau de la valvule mitrale.

4. Procédé selon la revendication 3, dans lequel l'identification comprend l'identification de l'emplacement de l'anneau de la valvule mitrale tel que représenté en coupe transversale dans une séquence d'images bidimensionnelles.

5. Procédé selon la revendication 3, dans lequel l'identification comprend l'identification de l'emplacement de l'anneau de la valvule mitrale tel que représenté dans une séquence d'images tridimensionnelles.

6. Procédé selon la revendication 2, dans lequel l'identification comprend en outre l'identification de l'emplacement du plan de la valvule mitrale sur les images; et
dans lequel l'affichage comprend en outre l'affichage sur une seule image du coeur des emplacements des positions successives du plan de la valvule mitrale.

7. Procédé selon la revendication 6, dans lequel l'identification comprend l'identification de l'emplacement du plan de la valvule mitrale tel que représenté en coupe transversale dans une séquence d'images bidimensionnelles.

8. Procédé selon la revendication 6, dans lequel l'identification comprend l'identification de l'emplacement du plan de la valvule mitrale tel que représenté dans une séquence d'images tridimensionnelles.

9. Procédé selon la revendication 2, dans lequel l'affichage comprend en outre l'affichage sur une seule image du coeur des emplacements ombrés différemment de façon distinctive des positions successives de la valvule mitrale.

10. Procédé selon la revendication 2, dans lequel l'affichage comprend en outre l'affichage sur une seule image du coeur des emplacements colorés différemment de façon distinctive des positions successives de la valvule mitrale.

11. Procédé selon la revendication 1, dans lequel l'affichage comprend en outre l'affichage sur chacune d'une pluralité d'images de la séquence de l'emplacement de la valvule mitrale sur cette image et de l'emplacement de la valvule mitrale sur les images précédentes de la séquence.

12. Procédé selon la revendication 11, comprenant en outre la réinitialisation de l'affichage d'une pluralité d'emplacements de la valvule mitrale suite à la fin de la phase ou des phases sélectionnée(s) du cycle cardiaque; et
la répétition des étapes d'acquisition, d'identification et d'affichage au cours d'un autre cycle cardiaque.

13. Procédé selon la revendication 1, dans lequel l'identification comprend en outre l'identification manuelle d'au moins un point de référence de la valvule mitrale sur une image du coeur acquise.

14. Procédé selon la revendication 1, dans lequel l'identification comprend en outre l'identification de l'emplacement de la valvule mitrale grâce au traitement par détection de bordure automatisé des images.

15. Procédé selon la revendication 1, dans lequel l'identification comprend en outre l'affichage sur une seule image du coeur des emplacements des positions successives de la valvule mitrale au cours de la séquence d'images en l'absence de l'affichage des positions successives d'au moins une partie de la paroi endocardique, moyennant quoi l'écho parasite de l'image est réduit.

16. Système d'imagerie diagnostique ultrasonore pour analyser la performance du coeur comprenant :
une sonde (410) possédant une pluralité (412) d'éléments transducteurs;
un processeur d'images (440) couplé à la sonde (410) qui sert à produire une séquence d'images en mode B ;
un processeur de détection de bordure automatisé (490) qui sert à identifier l'emplacement de la valvule mitrale dans une séquence d'images en mode B ;
un processeur d'affichage graphique (470), réactif à l'identification de l'emplacement de la valvule mitrale dans une séquence d'images en mode B, qui sert à produire un graphique décrivant les emplacements de la valvule mitrale dans une séquence d'images en mode B ; et
un affichage (480) réactif au processeur d'affichage graphique qui sert à afficher une image en mode B avec un graphique décrivant les emplacements de la valvule mitrale dans une séquence précédente d'images.

17. Système d'imagerie diagnostique ultrasonore selon la revendication 16, comprenant en outre une mémoire Cineloop (460), couplée au processeur de détection de bordure automatisé (490), qui stocke une séquence d'images en mode B.

18. Système d'imagerie diagnostique ultrasonore selon la revendication 16, dans lequel le processeur d'affichage graphique (470) comprend en outre un processeur qui sert à produire un graphique décrivant les emplacements de la valvule mitrale dans une séquence d'images en mode B grâce à des couleurs caractéristiques.

19. Système d'imagerie diagnostique ultrasonore selon la revendication 16, dans lequel le processeur de détection de bordure automatisé (490) comprend en outre un processeur qui sert à identifier l'emplacement du plan de la valvule mitrale dans une séquence d'images en mode B.

20. Système d'imagerie diagnostique ultrasonore pour analyser la performance du coeur comprenant :
une sonde (410) possédant une pluralité d'éléments transducteurs;
un processeur d'images (440) couplé à la sonde (410) qui sert à produire une séquence d'images en mode B;
une commande externe grâce à laquelle un utilisateur peut identifier l'emplacement de la valvule mitrale dans au moins l'une d'une séquence d'images en mode B;
un processeur d'affichage graphique (470), réactif à l'identification de l'emplacement de la valvule mitrale dans une séquence d'images en mode B, qui sert à produire un graphique décrivant les emplacements de la valvule mitrale dans une séquence d'images en mode B; et
un affichage (480) réactif au processeur d'affichage graphique (470) qui sert à afficher une image en mode B avec un graphique décrivant les emplacements de la valvule mitrale dans une séquence précédente d'images.

21. Système d'imagerie diagnostique ultrasonore selon la revendication 20, comprenant en outre un processeur de détection de bordure (490), réactif à la commande externe, qui sert à identifier l'emplacement de la valvule mitrale dans une séquence d'images en mode B.
